# EUROPÄISCHE PATENTSCHRIFT

(11) **EP 3 616 637 B1**
(45) Veröffentlichungstag und Bekanntmachung des Hinweises auf die Patenterteilung: **25.03.2026**
(21) Anmeldenummer: 18191442.5
(22) Anmeldetag: 29.08.2018
(51) Int. Cl.: A61B 18/14, A61B 18/00

(54) **MEDIZINISCHES INSTRUMENT**
MEDICAL INSTRUMENT
INSTRUMENT MÉDICAL

(43) Veröffentlichungstag der Anmeldung: 04.03.2020
(73) Patentinhaber: Erbe Elektromedizin GmbH, 72072 Tübingen (DE)
(72) Erfinder: Päsch, Markus, 72461 Albstadt (DE); Buntrock, Volker, 72762 Reutlingen (DE); Löser, David, 72108 Rottenburg am Neckar (DE); Schmidt, Stefanie, 72124 Pliezhausen (DE)
(74) Vertreter: Rüger Abel Patentanwälte PartGmbB

(56) Entgegenhaltungen:
- EP-A1- 2 959 854
- EP-A2- 2 156 802
- WO-A1-2017/190304
- US-A1- 2007 043 352
- US-A1- 2009 157 074
- US-A1- 2017 333 118

## Beschreibung

Die Erfindung betrifft ein medizinisches Instrument.

Aus EP 2 959 854 A1 ist ein chirurgisches Instrument bekannt, welches zwei Branchen aufweist, zwischen denen Gewebe gefasst werden kann, und welches Schneid- und Versiegelungselektroden aufweist, mit denen das gefasste Gewebe geschnitten und versiegelt werden kann. Weiter ist aus der US 2007/0043352 A1 ein Instrument zum Versiegeln von Gefäßen bekannt, dessen Werkzeugteil darauf eingerichtet ist, ein Gefäß zwischen zwei Versiegelungselektroden zu klemmen. Die Bewegung wenigstens einer der Versiegelungselektroden wird durch einen Handhebel bewirkt. Dem Handhebel ist ein elektrischer Schalter zugeordnet, der den zu den Versiegelungselektroden fließenden Strom steuert. Der Schalter wird erst dann von dem Betätigungshebel betätigt, wenn die Versiegelungselektroden das Gefäß fest zwischeneinander geklemmt haben, **d.h.,** wenn die entsprechenden Backen geschlossen sind. US 2009/157074 A1 wird auch als relevanter Stand der Technik angesehen.

Aufgabe der vorliegenden Erfindung ist es, ein verbessertes Konzept zur Aktivierung oder Deaktivierung von Funktionen eines medizinischen Instruments anzugeben.

Diese Aufgabe wird mit einem medizinischen Instrument nach Anspruch 1 gelöst:

Das erfindungsgemäße medizinische Instrument weist ein Gehäuse und eine Einrichtung zum Aktivieren oder Deaktivieren wenigstens einer Funktion des medizinischen Instruments auf. Die Einrichtung kann als Steuerungs- oder Schalteinrichtung bezeichnet werden. Die Einrichtung weist einen ersten Teil und einen zweiten Teil auf, welche relativ zu dem Gehäuse beweglich sind, wobei der zweite Teil dazu angeordnet und eingerichtet ist, in eine Wirkstellung bezüglich des ersten Teils und dabei bezüglich des Gehäuses bewegt zu werden, um dadurch die wenigstens eine Funktion des medizinischen Instruments zu aktivieren oder zu deaktivieren. In der Wirkstellung arbeiten der zweite Teil und der erste Teil zusammen, um die wenigstens eine Funktion zu aktivieren oder deaktivieren. Die wenigstens eine Funktion kann beispielsweise das Beaufschlagen von Elektroden des medizinischen Instruments mit elektrischer Leistung, insbesondere HF-Leistung, sein, wenn das medizinische Instrument Elektroden aufweist, um damit Gewebe zu versiegeln und/oder zu schneiden. Umgekehrt ist der erste Teil dazu angeordnet und eingerichtet, in eine Wirkstellung bezüglich des zweiten Teils und dabei bezüglich des Gehäuses bewegt zu werden, um dadurch die wenigstens eine Funktion des medizinischen Instruments zu aktivieren oder zu deaktivieren. Auch hierbei arbeiten erster Teil und zweiter Teil zusammen, um die wenigstens eine Funktion des medizinischen Instruments zu aktivieren oder zu deaktivieren. Bevorzugt braucht der jeweils andere Teil nicht unbedingt bewegt zu werden, um die wenigstens eine Funktion zu aktivieren oder deaktivieren, sondern der jeweils andere Teil kann in Ruhestellung verbleiben. Das medizinische Instrument ist bevorzugt ein chirurgisches Instrument, besonders bevorzugt ein HFchirurgisches Instrument, mit welchem mittels HF-Energie beispielsweise eine oder mehrere der Funktionen "Schneiden", "Versiegeln", "Devitalisieren" und/oder "Koagulieren" ausgeführt werden kann.

Bei dem erfindungsgemäßen medizinischen Instrument kann eine bestimmte Funktion durch wahlweises Bedienen eines Bedienabschnitts, mittels welchem der zweite Teil in die Wirkstellung bezüglich des ersten Teils bewegt wird, und eines weiteren Bedienabschnitts, mittels welchem der erste Teil bewegt wird, ausgelöst werden. Der Bedienabschnitt ist bevorzugt gesondert von dem weiteren Bedienabschnitt, beispielsweise ein gesondertes Bedienelement. Der Bedienabschnitt und der weitere Bedienabschnitt, welche zum Bedienen des zweiten Teils bzw. des ersten Teils bestimmt sind, sind vorzugsweise an dem Gehäuse angeordnet. Der Bedienabschnitt und der weitere Bedienabschnitt können beispielsweise Tasten oder Hebel sein, welche von dem Gehäuse getrennte Elemente sind, oder der Bedienabschnitt und der weitere Bedienabschnitt können beispielsweise Abschnitte des Gehäuses sein. Der Bedienabschnitt zum Bewegen des zweiten Teils in die Wirkstellung bezüglich des ersten Teils ist bevorzugt dazu eingerichtet, für die Bewegung des zweiten Teils relativ zu dem Gehäuse bewegt zu werden. Der weitere Bedienabschnitt zum Bewegen des ersten Teils in die Wirkstellung bezüglich des zweiten Teils ist bevorzugt dazu eingerichtet, für die Bewegung des ersten Teils relativ zu dem Gehäuse bewegt zu werden. Es wird beispielsweise nur ein Taster benötigt, um die Funktion wahlweise mit einem Bedienabschnitt oder einem weiteren Bedienabschnitt auszulösen. In Ausführungsbeispielen kann zum Auslösen derselben Funktion einerseits ein Element zum Drücken auf den Taster beispielsweise mittels des Bedienabschnitts gegen den Taster gedrückt werden und andererseits der Taster mittels des weiteren Bedienabschnitts gegen das Element zum Drücken auf den Taster bewegt werden. Gegenüber einer Lösung mit zwei Tastern hat die erfindungsgemäße Lösung einen geringeren Platzbedarf. Zudem ist die elektrische Kontaktierung vereinfacht. Insbesondere kann die Verkabelung vereinfacht sein. Die anderenfalls notwendigen zusätzlichen Kabel, etwa für einen weiteren Taster, würden das Risiko bergen, bewegliche Teile, **z.B.** eine Handgriffmechanik, soweit vorhanden, zu behindern und würden zu erhöhtem Entwicklungsaufwand führen, um diese gefahrenfrei zu fixieren. Gegenüber einer Lösung mit zwei Tastern können mit der erfindungsgemäßen Lösung die Kosten für die zusätzlichen Komponenten wie Taster, Platine, Kabel, Fixierung und die zusätzlichen Montageschritte eingespart werden, um niedrigere Fertigungskosten zu erhalten.

Das erfindungsgemäße medizinische Instrument kann mit einem oder mehreren der nachfolgend beschriebenen Merkmale oder gemäß einer oder mehrerer der nachfolgend beschriebenen Ausführungsformen vorteilhaft weitergebildet werden:

Das medizinische Instrument weist eine Leiterplatte auf, auf welcher der erste Teil oder der zweite Teil angeordnet ist, wobei die Leiterplatte in dem medizinischen Instrument beweglich gehalten ist. Die Leiterplatte kann insbesondere in einem Gehäuse des medizinischen Instruments, insbesondere einem Handgriffgehäuse, beweglich gehalten sein. Die Leiterplatte kann insbesondere schwenkbar und/oder kippbar und/oder gelenkig gehalten oder gelagert sein.

Der erste Teil oder der zweite Teil kann auf der Leiterplatte angeordnet sein, wobei die Leiterplatte einseitig bestückt ist. Die andere Seite der Leiterplatte kann, abgesehen von elektrischen Leitungen, frei von Bauelementen, wie beispielsweise Tastern, sein. Die Leiterplatte kann verglichen mit einer Leiterplatte mit beidseitiger Bestückung mit niedrigeren Fertigungskosten gefertigt werden. Die Leiterplatte kann zumindest nur auf einer Seite einen Taster aufweisen, welcher den ersten oder zweiten Teil bilden kann.

In anderen Ausführungsformen können beiden Seiten der Leiterplatte mit Bauelementen bestückt sein, wobei in diesem Ausführungsformen jedoch nur eine Seite der Leiterplatte mit einem Taster bestückt **ist.** Der Taster bildet **be**vorzugt den ersten Teil.

Der zweite Teil kann an einem Bedienabschnitt, beispielsweise einem Bedienelement, befestigt sein, welcher beweglich gelagert **ist.** Alternativ oder zusätzlich kann der erste Teil an einem weiteren Bedienabschnitt, beispielsweise einem weiteren Bedienelement, befestigt sein, welcher beweglich gelagert **ist.** Eine gesonderte bewegliche Lagerung des ersten Teils und/oder eine gesonderte bewegliche Lagerung des zweiten Teils kann damit entfallen.

In Ausführungsformen des medizinischen Instruments weist das medizinische Instrument einen Bedienabschnitt zum Bedienen des zweiten Teils, insbesondere zum Bewegen des zweiten Teils in die Wirkstellung bezüglich des ersten Teils, auf, wobei auf Grund des Bedienens des Bedienabschnitts eine weitere Funktion des medizinischen Instruments ausgeführt wird. Die weitere Funktion des medizinischen Instruments kann beispielsweise ein mechanisches Einwirken auf Gewebe sein, wie beispielsweise ein Fassen von Gewebe zwischen zwei Branchen. Der Bedienabschnitt kann rein mechanisch mit dem Werkzeug des medizinischen Instruments zum Ausführen der weiteren Funktion gekoppelt sein.

Der Bedienabschnitt ist vorzugsweise bedienbar so dass die weitere Funktion ausgeführt wird, ohne dass die Funktion aufgrund der Bedienung, insbesondere Bewegung, des Bedienabschnitts aktiviert oder deaktiviert wird. Das medizinische Instrument kann insbesondere derart eingerichtet sein, dass der Bedienabschnitt vom Benutzer bewegbar ist, wobei aufgrund eines ersten Abschnitts der Bewegung die weitere Funktion ausgeführt wird und erst aufgrund eines weiteren darauffolgenden Abschnitts der Bewegung die Funktion aktiviert oder deaktiviert wird.

Das medizinische Instrument ist vorzugsweise dazu eingerichtet, dem Benutzer mittels einer haptischen Rückmeldung zu vermitteln, dass bei weiterer Bewegung des Bedienabschnitts die wenigstens eine Funktion aktiviert oder deaktiviert wird. Die Rückmeldung ist für den Benutzer wahrnehmbar, z.B. akustisch wahrnehmbar oder optisch wahrnehmbar oder mit der Hand spürbar. Bevorzugt ist die Rückmeldung mit der Hand spürbar, mit welcher der Benutzer das Instrument bedient.

Bevorzugt weist das medizinische Instrument einen weiteren Bedienabschnitt zum Bedienen des ersten Teils, insbesondere zum Bewegen des ersten Teils in die Wirkstellung bezüglich des zweiten Teils auf. Besonders bevorzugt löst das Bedienen des weiteren Bedienabschnitts kein Ausführen einer weiteren Funktion des medizinischen Instruments aus.

Ein besonders einfacher Aufbau wird erhalten, wenn der weitere Bedienabschnitt ein Abschnitt eines Elements ist, welches Träger des ersten Teils oder einer Leiterplatte ist, welche den ersten Teil aufweist. Das Element bzw. der Träger ist vorzugsweise beweglich gehalten, insbesondere beweglich gelagert, beispielsweise schwenkbeweglich gelagert.

Eine besonders einfache Bedienbarkeit des medizinischen Instruments wird erhalten, wenn der Bedienabschnitt in proximale Richtung bewegbar ist, um die Funktion zu aktivieren oder deaktivieren und/oder um die weitere Funktion auszulösen, und wenn der weitere Bedienabschnitt in distale Richtung bewegbar ist, um die Funktion zu aktivieren oder deaktivieren. Alternativ wäre es beispielsweise auch möglich, wenn der Bedienabschnitt in distale Richtung bewegbar ist, um die Funktion zu aktivieren oder deaktivieren und/oder um die weitere Funktion auszulösen und der weitere Bedienabschnitt in proximale Richtung bewegbar ist, um die Funktion zu aktivieren oder deaktivieren. Der Bedienabschnitt und der weitere Bedienabschnitt sind vorzugsweise an unterschiedlichen Seiten des Instruments, insbesondere eines Handgriffs des Instruments angeordnet und/oder weisen in unterschiedliche Richtungen, beispielsweise distale Richtung und proximale Richtung. An dem distalen Ende des Instruments kann dieses einen Arbeitsabschnitt mit wenigstens einem Werkzeug aufweisen, welches die wenigstens eine Funktion ausführen kann. Der Bedienabschnitt ist vorzugsweise von dem Arbeitsabschnitt des Instruments weg bewegbar, um die Funktion und/oder die weitere Funktion zu aktivieren oder deaktivieren.

In bevorzugten Ausführungsformen ist der Bedienabschnitt zum Bedienen des zweiten Teils ein Abschnitt eines Elements, welches bezüglich des Elements, welches den zweiten Teil hält, beweglich ist. Beim Bedienen kann das Element, dessen Abschnitt der Bedienabschnitt zum Bedienen des zweiten Teils ist, gegen das Element, welches den zweiten Teil aufweist, gedrängt werden, um den zweiten Teil in die Wirkstellung bezüglich des ersten Teils zu bewegen, insbesondere den zweiten Teil gegen den ersten Teil zu drängen. Damit lässt sich realisieren, dass die Bewegung des Bedienabschnitts noch der Aktivierung einer weiteren Funktion dient, für welche eine andere oder weitere Bedienbewegung notwendig ist, als zum Bedienen des zweiten Teils. Selbstverständlich kann zwischen dem Element, dessen Abschnitt der Bedienabschnitt ist, und dem Element, welches den zweiten Teil, hält noch wenigstens ein beweglich gehaltenes Zwischenelement vorhanden sein, welches mittels des Elements, dessen Abschnitt der Bedienabschnitt ist, gegen das Element, welches den zweiten Teil hält, gedrängt wird, um den zweiten Teil in die Wirkstellung bezüglich des ersten Teils zu bewegen.

Das medizinische Instrument weist vorzugsweise eine weitere Einrichtung zum Aktivieren einer der Funktionen der wenigstens einen Funktion auf. Diese weitere Einrichtung ist vorzugsweise durch einen zusätzlichen Bedienabschnitt bedienbar. In Ausführungsbeispielen können die Funktionen "Versiegeln" und "Schneiden" beispielsweise mittels der Einrichtung aktiviert werden und mittels der weiteren Einrichtung kann nur die Funktion "Versiegeln" aktiviert werden. Beispielsweise kann die Einrichtung zum Aktivieren sowohl der Funktion "Versiegeln" als auch der Funktion "Schneiden" einen Taster aufweisen und die weitere Einrichtung kann zum Aktivieren nur der Funktion "Versiegeln" einen weiteren Taster aufweisen.

Der Bedienabschnitt zum Bewegen des zweiten Teils in die Wirkstellung bezüglich des ersten Teils ist zum Ausführen der weiteren Funktion des medizinischen Instruments bevorzugt mit einem Werkzeug mechanisch elastisch gekoppelt.

Weitere vorteilhafte Ausführungsformen und bevorzugte Merkmale ergeben sich aus den Unteransprüchen sowie nachfolgender Beschreibung und den Figuren. Es zeigen:
Figur 1 - ein Ausführungsbeispiel eines erfindungsgemäßen Instruments in schematisierter perspektivischer Gesamtdarstellung;
Figur 2 - den distalen Abschnitt des medizinischen Instruments gemäß Figur 1 mit der Gruppe von Werkzeugen in vergrößerter perspektivischer teilweise geschnittener Darstellung;
Figur 3 - einen seitlichen Blick in das zur Hälfte geöffnete Gehäuse des Handgriffs des beispielhaften medizinischen Instruments gemäß Figur 1.

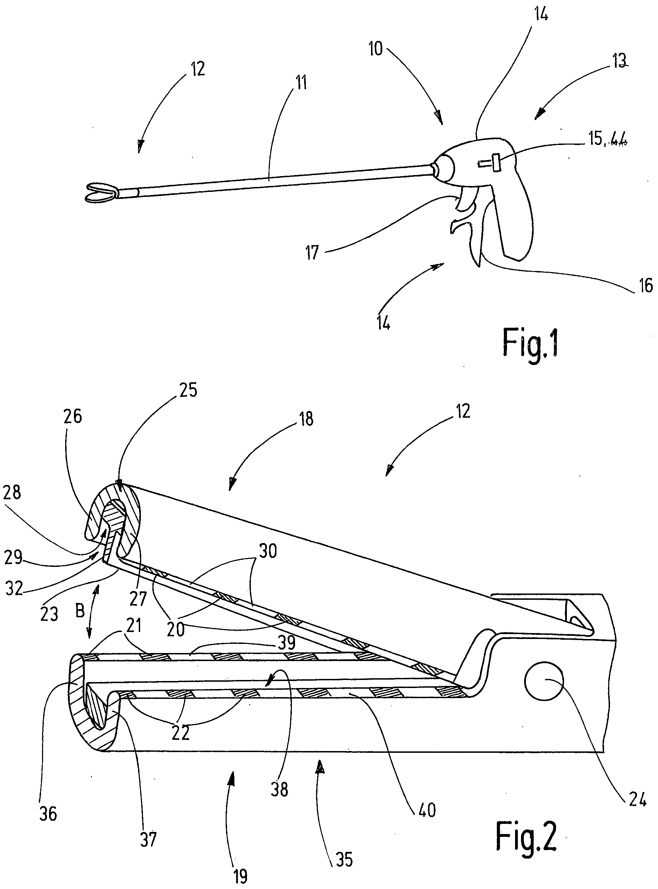

Bei dem erfindungsgemäßen medizinischen Instrument kann es sich insbesondere um ein chirurgisches Instrument 10, beispielsweise um ein solches wie schematisch und beispielhaft in den Figuren 1, 2 und 3 dargestellt, handeln. Das in den Figur 1, 2, 3 dargestellte medizinische Instrument 10 weist die Funktionen "Fassen von Gewebe", "Schneiden" und "Versiegeln" des Gewebes auf und kann als Versiegelungsinstrument bezeichnet werden. Entsprechend dient die Gruppe 12 von Werkzeugen des Instruments zum Versiegeln und ggfs. Durchtrennen von Gewebe, wobei im Gewebe enthaltene Gefäße und Lumen an dem entstehenden Gewebesaum geschlossen und somit abgedichtet sein sollen. Wenn auch die Erfindung im Folgenden an Hand eines Versiegelungsinstruments 10 erläutert wird, können auch andere medizinische Instrumente, welche genannte Funktionen nicht aufweisen und/oder andere Funktionen aufweisen, erfindungsgemäß weitergebildet werden.

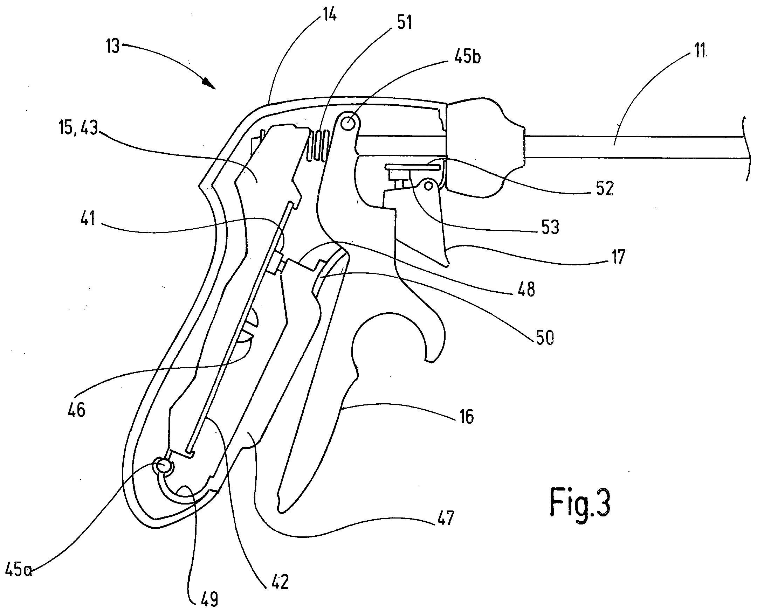

Das erfindungsgemäße medizinische Instrument 10 kann, wie in Figur 1 beispielhaft dargestellt, einen länglichen Schaft 11 aufweisen, der am distalen Ende eine Gruppe 12 von Werkzeugen trägt. Proximal ist der Schaft 11 mit einem Handgriffteil 13 verbunden, welches ein Gehäuse 14 aufweist. Zu dem Instrument 10 gehören Bedienelemente 15, 16, 17 (diese können auch als Betätigungselemente bezeichnet werden) zum Bedienen der Werkzeuge der Gruppe 12. Das erfindungsgemäße medizinische Instrument 10 weist beispielsweise ein erstes Bedienelement 15, ein zweites Bedienelement 16 und ein drittes Bedienelement 17 auf, welche beispielsweise wie dargestellt (Figuren 1 und 3) an dem Handgriffteil 13 angeordnet sein können. Die Werkzeuge der Gruppe 12 zum Ausführen der Funktionen können wie in Figur 2 beispielhaft dargestellt ausgeführt sein. Das dargestellte beispielhafte medizinische Instrument 10 weist ein Werkzeug zum Fassen von Gewebe mittels zweier Branchen 18, 19 auf, von denen mindestens eine beweglich gelagert ist. Als weiteres Werkzeug trägt jede Branche Elektroden 20 bzw. 21, 22 zum Versiegeln von Gewebe und eine Branche trägt eine Elektrode 23 zum Schneiden von Gewebe.

Das Werkzeug mit den Branchen 18, 19 zum Fassen des Gewebes sowie die Elektroden 20, 21, 22, 23 zum Schneiden und zum Versiegeln des gefassten Gewebes können beispielsweise wie in der EP 2 959 854 A1 erläutert ausgebildet sein und arbeiten.

Mindestens eine der Branchen 18, 19 des an dem Instrument 10 vorgesehenen Werkzeugs zum Fassen von Gewebe ist um die Schwenkachse 24 schwenkbar gelagert. In Figur 2 ist dies die obere Branche 18. Dabei kann je nach Anwendungsfall die erste Branche 18 oder die zweite Branche 19 oder es können beide Branchen 18, 19 um eine gemeinsame oder um unterschiedliche Schwenkachsen schwenkbar oder anderweitig beweglich gelagert sein, so dass die Branchen 18, 19 aufeinander zu und voneinander weg bewegt werden können.

Die erste Branche 18 weist einen Grundkörper 25 mit einem U-profilförmigen Querschnitt auf. Der Grundkörper 25 weist zwei zueinander parallele erste Versiegelungsbacken 26, 27, die elektrisch vorzugsweise miteinander verbunden sind, auf und die zwischen einander eine Nut 28 begrenzen. Diese erstreckt sich vorzugsweise über den größten Teil der Länge der ersten Branche 18 und dient der Aufnahme eines Schneidelektrodenträgers 29. Die Nut 28 ist von den Rändern der Versiegelungsbacken 26, 27 begrenzt, an denen die Versiegelungselektroden 20 ausgebildet sind. Auf Grund der Darstellung in Figur 2 sind nur die Versiegelungselektroden der einen ersten Versiegelungsbacke 27 sichtbar. Die Versiegelungselektroden 20 können elektrisch leitend mit dem Grundkörper 25 verbunden sein. Die Versiegelungselektroden 20 der beiden ersten Versiegelungsbacken 26, 27 bilden, wie aus Figur 2 hervorgeht, vorzugsweise eine Reihe voneinander beabstandeter leitender Einzelflächen, die durch isolierende Bereiche 30 voneinander getrennt sind.

Der Schneidelektrodenträger 29 weist eine Schneidelektrode 23 auf, die an seiner Stirnseite angeordnet ist. Die Schneidelektrode 23 sitzt dabei in einer Nut oder Ausnehmung des wandartigen Fortsatzes 32 des Schneidelektrodenträgers 29. Die Schneidelektrode 23 liegt mit einer Stirnfläche frei.

Die Länge des wandartigen Fortsatzes 32, gemessen von dem Fuß zu der Schneidelektrode 23 hin ist vorzugsweise so ausgeführt, dass die frei, der zweiten Branche 19 zugewandte Stirnfläche der Schneidelektrode 23 die Versiegelungsbacken 26, 27 überragt.

Die zweite Branche 19 weist ebenfalls einen Grundkörper 35 vorzugsweise aus elektrisch leitendem Material auf. Der Grundkörper 35 weist wiederum einen U-förmigen Querschnitt auf, bei dem zwischen zwei Versiegelungsbacken 36, 37 eine Nut 38 ausgebildet ist. An den elektrisch vorzugsweise miteinander verbundenen zweiten Versiegelungsbacken 36, 37 der zweiten Branche 19 sind die Versiegelungselektroden 21, 22 zwischen isolierenden Bereichen 39, 40 angeordnet.

Das erste Bedienelement 15 ist im dargestellten Ausführungsbeispiel eine mit dem Daumen zu bedienende Taste (Daumentaste) oder Hebel, welche seitlich aus dem Gehäuse 14 des Handgriffteils 13 herausschaut, wobei das erste Bedienelement 15 von proximal in distale Richtung zu drücken und dabei relativ zu dem Gehäuse 14 zu bewegen ist, um eine Elektrode, im dargestellten beispielhaften erfindungsgemäßen Instrument beispielsweise die Elektroden zum Schneiden 23 und Versiegeln 20, 21, 22 mit elektrischer Leistung zu beaufschlagen, um Gewebe mit diesen zu schneiden und zu versiegeln. Das zweite Bedienelement 16 ist bei dem dargestellten erfindungsgemäßen Instrument 10 unten an dem Gehäuse 14 des Handgriffteils 13 angeordnet und ein mit den Fingern zu bedienender Hebel, welcher an dem Gehäuse mittels des Schwenklagers 45b schwenkgelagert ist. Der Hebel 16 ist beispielsweise mechanisch mit einem Werkzeug des medizinischen Instruments gekoppelt, um mechanisch eine Handkraft auf das entsprechende Werkzeug zu übertragen. Im dargestellten Ausführungsbeispiel ist der Hebel 16 beispielsweise mechanisch mit der wenigstens einen schwenkbeweglichen Branche 18 gekoppelt, um die Branchen 18, 19 zu schließen, um Gewebe zu fassen, indem der Hebel 16 in proximale Richtung bewegt wird. Mittels des zweiten Bedienelements 16 lassen sich außerdem dieselben Funktionen auslösen, die auch mit dem ersten Bedienelement 15 ausgelöst werden können, wie weiter unten im Detail beschrieben ist. Das dritte Bedienelement 17, welches das medizinische Instrument 10 aufweisen kann, ist in dem dargestellten Ausführungsbeispiel eine vor dem zweiten Bedienelement 16 angeordnete Taste oder Hebel, um mit dem Zeigefinger bedient zu werden. Beim Zurückbewegen des dritten Bedienelements 17 in proximale Richtung mit dem Zeigefinger wird bevorzugt eine der Funktionen ausgelöst, welche durch Betätigen des ersten Bedienelements 15 oder durch Betätigen des zweiten Bedienelements 16 ausgelöst werden können. Beispielsweise kann durch Betätigen des dritten Bedienelements 17 die Funktion "Versiegeln" durch Beaufschlagen der Versiegelungselektroden 20, 21, 22 mit elektrischer Leistung ausgelöst werden, während die Schneidelektrode 23 nicht derart mit dem dritten Bedienelement 17 gekoppelt ist, dass durch Betätigen des dritten Bedienelements 17 die Schneidelektrode 23 mit elektrischer Leistung beaufschlagt werden würde.

Figur 3 veranschaulicht eine ausschnittsweise Seitenansicht auf das Ausführungsbeispiel des erfindungsgemäßen Instruments 10 gemäß Figur 1 (der distale Arbeitsabschnitt des Instruments 10 mit der Gruppe 12 von Werkzeugen ist nicht dargestellt), in welcher das Innere des Gehäuses 14 durch das seitlich geöffnete Gehäuse 14 erkennbar ist. Das Instrument 10 weist eine Aktivierungseinrichtung zum Aktivieren der Funktionen "Schneiden" und "Versiegeln" auf, zu welcher als erster Teil ein Taster 41 gehört, welcher von einer Platine 42 (Leiterplatte) getragen wird. Der Taster 41 ist mit einer Steuerung (nicht dargestellt) zur Beaufschlagung der Elektroden 20, 21, 22, 23 mit HF-Leistung zum "Schneiden" und "Versiegeln" verbunden, so dass bei entsprechendem Schaltzustand der Kontakte des Tasters 41 die entsprechenden Elektroden 20, 21, 22, 23 mit elektrischer Leistung beaufschlagt werden.

Die Platine 42 ist bevorzugt nur einseitig mit elektrischen Bauelementen bestückt, insbesondere ist nur auf einer Seite der Platine 42 ein Taster 41, angeordnet. Die Platine 42 ist bevorzugt auf dem ersten Bedienelement 15 befestigt. Das erste Bedienelement 15 weist einen in dem Gehäuse 14 angeordneten Halteabschnitt 43 und einen außerhalb des Gehäuses angeordneten Bedienabschnitt 44 (s. Figur 1) auf. Das erste Bedienelement 15 ist beweglich, insbesondere schwenkbeweglich in dem Gehäuse 14 gehalten. Das erste Bedienelement 15 ist in dem Ausführungsbeispiel in dem Gehäuse 14 an einem mit dem Gehäuse 14 verbundenen Schwenklager 45a schwenkgelagert. In der dargestellten Ausführungsform ist die Platine 42 auf dem ersten Bedienelement 15 beispielsweise mittels eines Schnapp-Rast-Abschnitts 46, welcher in eine Öffnung (nicht dargestellt) in der Platine 42 eingreift, befestigt. Die Platine 42 ist folglich mittels des ersten Bedienelements 15 beweglich in dem Gehäuse 14 gelagert. Eine Bewegung des ersten Bedienelements 15 in proximale Richtung kann durch einen Anschlagabschnitt (nicht dargestellt) begrenzt sein. Der Anschlagabschnitt kann beispielsweise an dem Gehäuse 14 befestigt oder angeformt sein. Das erste Bedienelement 15 kann in proximaler Richtung federnd gegen den Anschlagabschnitt vorgespannt sein. Figur 3 zeigt das erste Bedienelement 15 in seiner unbetätigten Ruhestellung.

Der Platine 42 und dem Taster 41 gegenüberliegend ist ein Halteelement 47 angeordnet, welches einen Betätigungsabschnitt 48 aufweist. Der Betätigungsabschnitt 48 ist durch einen Betätigungsabschnitt 48 des Halteelements 47 gebildet. Das Halteelement 47 ist in dem Gehäuse 14 beweglich gehalten. Dazu weist das Halteelement 47 einen federnden Abschnitt 49 auf, welcher an derselben Stelle mit dem Gehäuse 14 verbunden ist, an welcher auch das erste Bedienelement 15 mit dem Gehäuse 14 verbunden ist. Das Halteelement 47 ist dazu eingerichtet und angeordnet, so dass der Betätigungsabschnitt 48 bezüglich des Tasters 41 in eine Wirkstellung bewegt werden kann. In der dargestellten Ausführungsform ist der Betätigungsabschnitt 48 gegen den Taster 41 drängbar, um einen elektrischen Kontakt des Tasters 41 zu öffnen oder zu schließen, um das Schneiden mittels der Schneideelektrode 23 und das Versiegeln mittels der Versiegelungselektroden 20, 21, 22 zu aktivieren. Das Drängen des Betätigungsabschnitts 48 gegen den Taster 41 erfolgt durch Bewegen des zweiten Bedienelements 16 in proximale Richtung, welches dadurch gegen das Halteelement 47 gedrängt wird, welches in seiner Ruhestellung (diese ist in Figur 3 dargestellt) aus dem Gehäuse 14 herausschauen kann, indem es über die Kontur des Gehäuses übersteht. Die Bewegung des Halteelements 47 in distale Richtung kann durch einen Anschlagabschnitt 50 begrenzt sein, welcher an dem Gehäuse 14 befestigt oder angeformt sein kann. Das Halteelement 47 kann in seiner Ruhestellung federnd gegen den Anschlagabschnitt 50 vorgespannt gehalten sein.

Das zweite Bedienelement 16 und das Halteelement 47 sind, wie dargestellt, bevorzugt voneinander gesonderte Elemente. Das zweite Bedienelement 16 ist derart angeordnet, insbesondere von dem Halteelement 47 beabstandet, dass das Bedienen des zweiten Bedienelements 16 aus der distalen Ruhestellung (das zweite Bedienelement 16 kann in dieser gegen einen Anschlag vorgespannt sein, z.B. mittels einer Druckfeder, welche weiter unten beschrieben ist) in proximale Richtung zunächst zu einer Schließbewegung der beweglichen Branche 18 führt, ohne dass die Funktionen "Schneiden" und "Versiegeln" aktiviert würden, und erst im weiteren Bewegungsverlauf des zweiten Bedienelements 16 zur Aktivierung der Funktionen "Schneiden" und "Versiegeln" durch Betätigen des Tasters 41 über das Halteelement 47 führt.

Das erfindungsgemäße Instrument 10 kann dazu eingerichtet sein, dem Benutzer beim Bedienen des zweiten Bedienelements 16 eine mit der Hand spürbare Rückmeldung darüber zu geben, dass bei weiterer Bewegung des zweiten Bedienelements 16 eine Aktivierung der Funktion "Schneiden" und "Versiegeln" erfolgt. Dies kann beispielsweise mittels einer Einrichtung (nicht dargestellt) erfolgen, die dazu führt, dass der Benutzer zum Bewegen des zweiten Bedienelements 16 in Richtung zu dem Halteelement 47 in einer bestimmten Stellung des zweiten Bedienelements 16 durch den Benutzer mit der Hand spürbar mehr Kraft aufwenden muss, um das zweite Bedienelement 16 weiter in Richtung zu dem Halteelement 47 bzw. in proximale Richtung zu bewegen.

Das zweite Bedienelement 16 ist mit einem Werkzeug des medizinischen Instruments, beispielsweise mit der schwenkbeweglichen Branche 18 des dargestellten erfindungsgemäßen Instruments 10 vorzugsweise nicht steif, sondern mechanisch elastisch gekoppelt, insbesondere mittels eines Federelements, wie beispielsweise einer Druckfeder 51, um die Kraft auf das Werkzeug, beispielsweise zum Betätigen der Branche 18, zu übertragen. Die elastische Kopplung, beispielsweise das Federelement 51, ist dazu eingerichtet und bestimmt, die Steifigkeit der Übertragungsstrecke zur Übertragung der Handkraft auf die wenigstens eine schwenkbewegliche Branche im Wesentlichen zu bestimmen. Das Federelement 51 zeichnet sich dadurch aus, dass es eine kleinere Federkonstante aufweist als die anderen Übertragungsglieder zur Übertragung der Bewegung des zweiten Bedienelements 16 auf das Werkzeug und soweit vorgespannt ist dass die gewünschte Klemmkraft zwischen den Branchen 18, 19 erreicht wird, bevor sie sich verformt. Die Bewegung des zweiten Bedienelements 16 wird in eine proximale Bewegung des Federelements 51 umgesetzt, welche wiederum in eine Bewegung des Werkzeugs, beispielsweise der Branche 18, umgesetzt wird. Die elastische Verformung des Federelements 51 nach Erreichen der gewünschten Klemmkraft kann beispielsweise mittels einer zug- und/oder drucksteifen Zug- und/oder Druckstange erreicht werden. Dies führt zu einer Kraftbegrenzung bzw. zu einer Begrenzung der Übertragung der Kraft vom zweiten Bedienelement 16 auf das Werkzeug. Die mechanisch-elastische Kopplung führt umgekehrt dazu, das Werkzeug insofern von dem Bedienelement 16 zu entkoppeln, dass das Bedienelement 16 auch dann in die Stellung zum Aktivieren oder Deaktivieren der Funktion durch Betätigen des Tasters 41 über das Halteelement 47 bringbar ist, wenn die Bewegung des Werkzeugs, beispielsweise der Branche 18, über eine bestimmte Stellung hinaus behindert wird, beispielsweise durch Gewebe, das zwischen den Branchen 18, 19 gefasst ist, so dass sich die Branchen 18, 19 nicht mehr weiter schließen lassen, wenn das zweite Bedienelement 16 betätigt wird. Aufgrund der elastischen Kopplung lässt sich das Bedienelement 16 jedoch unter elastischer Verformung des Federelements 51 in die Stellung zum Aktivieren oder Deaktivieren der Funktionen "Schneiden" und "Versiegeln" bewegen.

Erfindungsgemäß hat der Benutzer eine weitere Möglichkeit zur Aktivierung der Funktionen "Versiegeln" und "Schneiden". Denn der Taster 41, ist mittels des ersten Bedienelements 15 gegen den Betätigungsabschnitt 48, im Beispiel gegen den Betätigungsabschnitt des Halteelements 47, drängbar. Damit wird der Taster 41 in eine Wirkstellung bezüglich des Betätigungsabschnitts 48 bewegt und zwar in einer Richtung, welche der Bewegungsrichtung des Betätigungsabschnitts 48 zum Bewegen des Betätigungsabschnitts 48 in eine Wirkstellung bezüglich des Tasters 41 entgegengesetzt ist. Wenn der Taster 41 mittels des ersten Bedienelements 15 gegen den Betätigungsabschnitt 48 gedrängt wird, stützt sich das Halteelement 47 an dem Anschlagabschnitt 50 ab. Während im dargestellten Ausführungsbeispiel ein mechanischer Kontakt des Tasters 41 und dem Betätigungsabschnitt 48 notwendig ist, so dass der Taster 41 einen elektrischen Kontakt herstellt oder trennt, kann der Taster 41 beispielsweise auch ein kapazitiver Sensor oder ein induktiver Sensor sein, so dass nur erforderlich ist, dass der Betätigungsabschnitt 48 in eine bestimmte Stellung (Wirkstellung) bezüglich des Tasters 41 bewegt wird, bzw. dass der Taster 41 in eine bestimmte Stellung (Wirkstellung) bezüglich des Betätigungsabschnitts 48 bewegt wird, um das Herstellen oder Trennen einer elektrischen Verbindung zum Aktivieren oder Deaktivieren durch den ersten Teil 41 beispielsweise kapazitiv oder induktiv auszulösen, ohne dass eine Krafteinwirkung des Betätigungsabschnitts 48 auf den Taster 41 oder eine Krafteinwirkung des Tasters 41 auf das Halteelement 47 unbedingt notwendig wäre, so dass der Taster 41 die elektrische Verbindung herstellt oder trennt.

Das Instrument 10 weist vorzugsweise eine weitere Aktivierungseinrichtung auf, mit welcher eine der Funktionen, z.B. "Versiegeln" aktivierbar ist, welche Funktionen mittels des ersten und zweiten Bedienelements 15, 16 aktiviert werden können. Bevorzugt ist ein Aktivieren der wenigstens einen anderen Funktion (z.B. "Schneiden"), welche durch Bedienen des ersten Bedienelements 15 aktiviert werden kann, mittels der weiteren Aktivierungseinrichtung nicht möglich. Die weitere Aktivierungseinrichtung kann beispielsweise, wie dargestellt, eine weitere Leiterplatte 52 mit einem weiteren Taster 53 aufweisen, wobei das dritte Bedienelement 17 durch Bewegen des dritten Bedienelements 17 in proximale Richtung in Wirkstellung bezüglich des weiteren Tasters 53 bewegbar ist, um auf den weiteren Taster 53 zu drücken, so dass der weitere Taster 53 aufgrund dessen die Funktion auslöst.

Das erfindungsgemäße medizinische Instrument 10 arbeitet beispielsweise wie folgt:
Zur Versiegelung und Trennung von Geweben, insbesondere Hohlgefäßen oder Hohlgefäße enthaltenden Geweben soll dieses zwischen den Branchen 18, 19 gefasst werden. Durch entsprechende Betätigung des zweiten Bedienelements 16 wird die bewegliche Branche 18 so auf die andere Branche 19 zu bewegt, dass biologisches Gewebe zwischen den Branchen 18, 19 gefasst wird. Der Benutzer zieht dazu den Hebel 16, der das zweite Bedienelement 16 bildet zu sich heran, also in proximale Richtung. Das Instrument 10 kann dem Benutzer eine mit der Hand, mit welcher der Benutzer das Instrument 10 hält, spürbare Rückmeldung geben, dass beim weiteren Zurückziehen des Hebels 16 eine Aktivierung der Funktionen "Versiegeln" und "Schneiden" erfolgt. Zur Gewebeversiegelung wird zwischen den Branchen 18, 19 eine elektrische Spannung, vorzugsweise eine hochfrequente Wechselspannung wirksam, so dass zwischen den Elektroden 20 der ersten Branche 18 und den Elektroden 21, 22 der zweiten Branche 19 ein elektrischer Strom durch das biologische Gewebe fließt, um dieses zu erwärmen und eine Fusion des gefassten Gewebes herbeizuführen. Zeitgleich wird die Schneidelektrode 23 aktiviert. Dieser wird auch eine elektrische Spannung, vorzugsweise eine HF-Spannung zugeführt, deren Bezugspotential auf einer der Branchen 18, 19 vorzugsweise auf der zweiten Branche 19, **d.h.** den Versiegelungselektroden 21, 22 liegt. Die Stromdichte an der Stirnfläche der Schneidelektrode 23 ist so hoch, dass das biologische Gewebe zügig durchtrennt wird.

Der Benutzer kann die Elektroden 23, 20, 21, 22 zum Schneiden und Versiegeln auch mit HF-Leistung speisen, ohne den Hebel 16, welcher das zweite Bedienelement 16 bildet, in proximale Richtung zu ziehen, indem der Benutzer auf das erste Bedienelement 15 drückt. Damit können ein Gewebeschnitt und/oder eine Koagulation auch bei offenen Branchen 18, 19 erfolgen, **z.B.** um große Gefäße zu schneiden. Auch möglich ist, die Elektroden 23, 20, 21, 22 zum Schneiden und zur Versiegelung mittels des ersten Bedienelements 15 mit HF-Leistung zu versorgen, wenn die Branchen 18, 19 teilgeschlossen sind und das zweite Bedienelement 16 in einer Stellung ist, so dass das Halteelement 47 auf Grund des Abstands zwischen Halteelement 47 und zweitem Bedienelement 16 noch nicht aktivierend gegen den Taster 41 gedrückt wird.

Der Benutzer kann zudem bei offenen oder teilgeschlossenen Branchen 18, 19 die Versiegelung mittels des dritten Bedienelements 17 aktivieren, welches bei Betätigung auf den weiteren Taster 53 einwirkt.

Erfindungsgemäß wird ein medizinisches Instrument 10 angegeben, welches eine Einrichtung zum Aktivieren oder Deaktivieren einer Funktion des medizinischen Instruments aufweist. Die Einrichtung weist einen Taster 41 und einen Betätigungsabschnitt 48 auf, wobei der Betätigungsabschnitt 48 dazu angeordnet und eingerichtet ist, in eine Wirkstellung bezüglich des Tasters 41 bewegt zu werden, um dadurch wenigstens eine Funktion des medizinischen Instruments 10 zu aktivieren oder deaktivieren, und wobei der Taster 41 dazu angeordnet und eingerichtet ist, in eine Wirkstellung bezüglich des Betätigungsabschnitts 48 bewegt zu werden, um dadurch die wenigstens eine Funktion des medizinischen Instruments 10 zu aktivieren oder zu deaktivieren. Das medizinische Instrument 10 weist zum Bewegen des Betätigungsabschnitts 48 in die Wirkstellung vorzugsweise ein zweites Bedienelement 16 auf, welches einen Bedienabschnitt bereitstellt, und weist zum Bewegen des Tasters 41 in die Wirkstellung vorzugsweise ein erstes Bedienelement 15 auf, welches einen weiteren Bedienabschnitt bereitstellt. Das erste Bedienelement 15 ist zur Aktivierung der Funktion vorzugsweise in eine Richtung bewegbar, die der Richtung in welche das zweite Bedienelement 16 zur Aktivierung der Funktion bewegbar ist, entgegengesetzt ist. Dadurch ist die Funktion beispielsweise durch Bewegung eines Bedienelements 15 von hinten nach vorne, **d.h.** in distale Richtung, und durch Bewegung eines anderen Bedienelements 16 in umgekehrte Richtung, von vorne nach hinten, **d.h.** in proximale Richtung aktivierbar. Ein Umlenken der Kraft von einem Betätigungselement 15, 16 zum Betätigen des ersten Teils oder des zweiten Teils in entgegengesetzte Richtung ist damit nicht unbedingt erforderlich.

### Bezugszeichenliste:

| | |
|---|---|
| 10 | Instrument |
| 11 | Schaft |
| 12 | Gruppe von Werkzeugen |
| 13 | Handgriffteil |
| 14 | Gehäuse |
| 15 | erstes Bedienelement |
| 16 | zweites Bedienelement |
| 17 | drittes Bedienelement |
| 18 | Erste Branche |
| 19 | Zweite Branche |
| 20, 21, 22 | Versiegelungselektrode |
| 23 | Schneidelektrode |
| 24 | Schwenkachse |
| 25 | Grundkörper |
| 26 | Versiegelungsbacke |
| 27 | Versiegelungsbacke |
| 28 | Nut |
| 29 | Schneidelektrodenträger |
| 30 | isolierende Bereiche |
| 32 | Fortsatz |
| 35 | Grundkörper |
| 36 | Versiegelungsbacke |
| 37 | Versiegelungsbacke |
| 38 | Nut |
| 39 | Isolierender Bereich |
| 40 | Isolierender Bereich |
| 41 | Taster |
| 42 | Platine/Leiterplatte |
| 43 | Halteabschnitt |
| 44 | Bedienabschnitt |
| 45a | Schwenklager |
| 45b | Schwenklager |
| 46 | Schnapp-Rast-Abschnitt |
| 47 | Halteelement |
| 48 | Betätigungsabschnitt |
| 49 | federnder Abschnitt |
| 50 | Anschlagabschnitt |
| 51 | Druckfeder |
| 52 | weitere Leiterplatte |
| 53 | weiterer Taster |

## Patentansprüche

1. Medizinisches Instrument (10) mit einem Gehäuse (14) und mit einer Einrichtung zum Aktivieren oder Deaktivieren einer Funktion des medizinischen Instruments, wobei die Einrichtung einen Taster (41) und einen Betätigungsabschnitt (48) aufweist, wobei der Betätigungsabschnitt (48) dazu angeordnet und eingerichtet ist, in eine Wirkstellung bezüglich des Tasters (41) und dabei bezüglich des Gehäuses (14) bewegt zu werden, um dadurch wenigstens eine Funktion des medizinischen Instruments (10) zu aktivieren oder deaktivieren, und wobei der Taster (41) dazu angeordnet und eingerichtet ist, in eine Wirkstellung bezüglich des Betätigungsabschnitts (48) und dabei bezüglich des Gehäuses (14) bewegt zu werden, um dadurch die wenigstens eine Funktion des medizinischen Instruments (10) zu aktivieren oder zu deaktivieren,
wobei ein erstes Bedienelement (15) mit einem in dem Gehäuse (14) angeordneten Halteabschnitt (43) und einem außerhalb des Gehäuses vorgesehenen Bedienabschnitt (44) vorgesehen ist, wobei das Bedienelement (15) in dem Gehäuse (14) beweglich gehalten ist,
wobei der Taster (41) auf einer Leiterplatte (42) angeordnet ist, welche mittels des ersten Bedienelements (15) in dem Gehäuse (14) beweglich gehalten ist,
wobei die Leiterplatte (42) einseitig mit dem Taster (41) bestückt ist,
wobei der Leiterplatte (42) und dem Taster (41) gegenüberliegend ein an dem Gehäuse (14) beweglich gehaltenes Halteelement (47) angeordnet ist, welches den Betätigungsabschnitt (48) aufweist.

2. Medizinisches Instrument (10) nach einem der vorstehenden Ansprüche, wobei das medizinische Instrument (10) einen Bedienabschnitt (16) zum Bewegen des Betätigungsabschnitts (48) in die Wirkstellung bezüglich des Tasters (41) aufweist, wobei auf Grund des Bedienens des Bedienabschnitts (16) eine weitere Funktion des medizinischen Instruments (10) ausgeführt wird.

3. Medizinisches Instrument (10) nach Anspruch 2, wobei der Bedienabschnitt (16) bedienbar ist, so dass die weitere Funktion ausgeführt wird, ohne dass die Funktion auf Grund der Bedienung des Bedienabschnitts (16) aktiviert oder deaktiviert wird.

4. Medizinisches Instrument (10) nach Anspruch 3, wobei das medizinische Instrument (10) dazu eingerichtet ist, dem Benutzer, beispielsweise mit der Bedienhand spürbar, zu vermitteln, dass bei weiterer Bewegung des Bedienabschnitts (16) die wenigstens eine Funktion aktiviert oder deaktiviert wird.

5. Medizinisches Instrument (10) nach einem der vorstehenden Ansprüche, wobei das medizinische Instrument (10) einen weiteren Bedienabschnitt (44) zum Bewegen des Tasters (41) in die Wirkstellung bezüglich des zweiten Teils (48) aufweist, wobei das Bedienen des weiteren Bedienabschnitts (44) kein Ausführen einer weiteren Funktion des medizinischen Instruments (10) auslöst.

6. Medizinisches Instrument (10) nach einem der vorstehenden Ansprüche 2 bis 5, wobei der Bedienabschnitt (16) in proximale Richtung bewegbar ist, um die Funktion zu aktivieren oder deaktivieren und/oder um die weitere Funktion auszulösen und wobei der weitere Bedienabschnitt (44) in distale Richtung bewegbar ist, um die Funktion zu aktivieren oder deaktivieren.

7. Medizinisches Instrument (10) nach Anspruch 2, wobei der Bedienabschnitt (16) zum Bedienen des zweiten Teils (48) ein Abschnitt eines Elements (16) ist, welches bezüglich des Halteelements (47), welches das Betätigungselement (48) aufweist, bewegbar ist.

8. Medizinisches Instrument (10) nach einem der vorstehenden Ansprüche, wobei das medizinische Instrument (10) eine weitere Einrichtung (53, 17) zum Aktivieren einer Funktion der wenigstens einen Funktion aufweist.

## Claims

1. A medical instrument (10) with a housing (14) and with a device for activating or deactivating a function of the medical instrument, wherein the device comprises a button (41) and an actuating portion (48), wherein the actuating portion (48) is arranged and configured to be moved into an operative position with respect to the button (41) and thereby with respect to the housing (14) in order to thereby activate or deactivate at least one function of the medical instrument (10), and wherein the button (41) is arranged and configured to be moved into an operative position with respect to the actuating portion (48) and thereby with respect to the housing (14) in order to thereby activate or deactivate the at least one function of the medical instrument (10),
wherein a first control element (15) is provided with a holding portion (43) arranged in the housing (14) and a control portion (44) provided outside the housing, wherein the control element (15) is held movably in the housing (14),
wherein the button (41) is arranged on a circuit board (42), which is held movably in the housing (14) by means of the first control element (15),
wherein the circuit board (42) is equipped with the button (41) on one side,
wherein a holding element (47) which is held movably on the housing (14) is arranged opposite the circuit board (42) and the button (41), which holding element (47) has the actuating portion (48).

2. The medical instrument (10) according to anyone of the preceding claims, wherein the medical instrument (10) has a control portion (16) for moving the actuating portion (48) into the operative position with respect to the button (41), wherein a further function of the medical instrument (10) is performed based on the control of the control portion (16).

3. The medical instrument (10) according to claim 2, wherein the control portion (16) is controllable so that the further function is performed without the function being activated or deactivated due to the control of the control portion (16).

4. The medical instrument (10) according to claim 3, wherein the medical instrument (10) is configured to convey to the user, for example perceptibly with the controlling hand, that upon further movement of the control portion (16) the at least one function is activated or deactivated.

5. The medical instrument (10) according to anyone of the preceding claims, wherein the medical instrument (10) comprises a further control portion (44) for moving the button (41) into the operative position with respect to the second part (48), wherein the control of the further control portion (44) does not trigger the execution of any further function of the medical instrument (10).

6. The medical instrument (10) according to anyone of the preceding claims 2 to 5, wherein the control portion (16) is movable in a proximal direction to activate or deactivate the function and/or to trigger the further function, and wherein the further control portion (44) is movable in a distal direction to activate or deactivate the function.

7. The medical instrument (10) according to claim 2, wherein the control portion (16) for controlling the second part (48) is a portion of an element (16) which is movable with respect to the holding member (47) which comprises the actuating element (48).

8. The medical instrument (10) according to anyone of the preceding claims, wherein the medical instrument (10) comprises a further device (53, 17) for activating a function of the at least one function.

## Revendications

1. Instrument médical (10) comprenant un boîtier (14) et comprenant un dispositif pour activer ou désactiver une fonction de l'instrument médical, le dispositif présentant un bouton-poussoir (41) et une partie d'actionnement (48), la partie d'actionnement (48) étant disposée et agencée de manière à être déplacée dans une position active par rapport au bouton-poussoir (41) et à être de ce fait déplacée par rapport au boîtier (14), aux fins d'activer ou de désactiver ainsi au moins une fonction de l'instrument médical (10), et le bouton-poussoir (41) étant disposé et agencé de manière à être déplacé dans une position active par rapport à la partie d'actionnement (48) et à être de ce fait déplacé par rapport au boîtier (14), aux fins d'activer ou de désactiver ainsi la fonction, au nombre d'au moins une, de l'instrument médical (10),
dans lequel il est prévu un premier élément de commande (15) comportant une partie de maintien (43), disposée dans le boîtier (14), et une partie de commande (44) disposée à l'extérieur du boîtier, l'élément de commande (15) étant tenu de façon mobile dans le boîtier (14),
dans lequel le bouton-poussoir (41) est disposé sur une carte de circuit imprimé (42) qui est tenue de façon mobile dans le boîtier (14) au moyen du premier élément de commande (15),
dans lequel la carte de circuit imprimé (42) est équipée sur une face avec le bouton-poussoir (41),
dans lequel un élément de maintien (47) est disposé de façon mobile sur le boîtier (14), en vis-à-vis de la carte de circuit imprimé (42) et du bouton-poussoir (41), et comporte la partie d'actionnement (48).

2. Instrument médical (10) selon une des revendications précédentes, dans lequel l'instrument médical (10) présente une partie de commande (16) destinée à déplacer la partie d'actionnement (48) dans la position active par rapport au bouton-poussoir (41), sachant que la manœuvre de la partie de commande (16) a pour effet d'exécuter une fonction supplémentaire de l'instrument médical (10).

3. Instrument médical (10) selon la revendication 2, dans lequel la partie de commande (16) peut être manœuvrée de manière à ce que la fonction supplémentaire soit exécutée sans que la fonction soit activée ou désactivée du fait de la manœuvre de la partie de commande (16).

4. Instrument médical (10) selon la revendication 3, dans lequel l'instrument médical (10) est agencé pour indiquer à l'utilisateur, par exemple de manière perceptible par la main effectuant la manœuvre, qu'en poursuivant le déplacement de la partie de manœuvre (16), la fonction, au nombre d'au moins une, est activée ou désactivée.

5. Instrument médical (10) selon une des revendications précédentes, dans lequel l'instrument médical (10) présente une partie de commande (44) supplémentaire destinée à déplacer le bouton-poussoir (41) dans la position active par rapport à la deuxième partie (48), la manœuvre de la partie de commande (44) supplémentaire ne déclenchant pas l'exécution d'une fonction supplémentaire de l'instrument médical (10).

6. Instrument médical (10) selon une des revendications précédentes 2 à 5, dans lequel la partie de commande (16) peut être déplacée dans la direction proximale pour activer ou désactiver la fonction et/ou pour déclencher la fonction supplémentaire, et dans lequel la partie de commande (44) supplémentaire peut être déplacée dans la direction distale pour activer ou désactiver la fonction.

7. Instrument médical (10) selon la revendication 2, dans lequel la partie de commande (16) destinée à manœuvrer la deuxième partie (48) est une portion d'un élément (16) qui peut être déplacé par rapport à l'élément de maintien (47) qui comporte l'élément d'actionnement (48).

8. Instrument médical (10) selon une des revendications précédentes, dans lequel l'instrument médical (10) présente un dispositif (53, 17) supplémentaire pour activer une fonction de la fonction, au nombre d'au moins une.
